# EUROPEAN PATENT APPLICATION

(11) **EP 1 860 107 A1**
(43) Date of publication of application: **28.11.2007**
(21) Application number: 06702560.1
(22) Date of filing: 13.01.2006
(51) Int. Cl.: C07D 487/22, A61K 31/409, A61K 41/00, A61P 35/00

(54) **PHOTOSENSITIVE COMPOUND**

(30) Priority: 14.01.2005 JP 2005008341
(71) Applicant: Hamamatsu Foundation for Science and Technology Promotion, Hamamatsu-shi, Shizuoka 432-8561 (JP)
(72) Inventor: KANAYAMA, Naohiro c/o Hamamatsu University School of Medicine, Shizuoka 431-3192 (JP); HORIUCHI, Kentaro c/o Hamamatsu University School of Medicine, Shizuoka 431-3192 (JP); OZAWA, Hidechika c/o Hamamatsu University School of Medicine, Shizuoka 431-3192 (JP); HIRANO, Toru c/o Hamamatsu University School of Medicine, Shizuoka 431-3192 (JP); KOHNO, Eiji c/o Hamamatsu University School of Medicine, Shizuoka 431-3192 (JP)
(74) Representative: Becker, Philippe
(86) International application number: PCT/JP2006/300328
(87) International publication number: WO 2006/075678

(57) **Abstract**

To provide a novel photosensitive compound which is useful for a photodynamic therapy, an agent for the photodynamic therapy containing the same and a method for producing the same. A compound represented by the following formula 1 or pharmaceutically acceptable salts thereof.

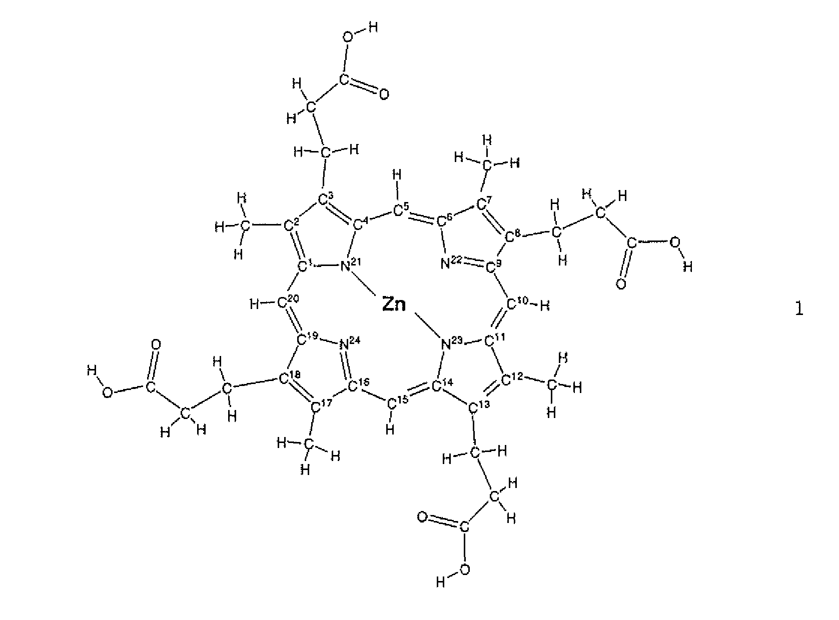

## Description

### Technical Field

The present invention relates to a novel photosensitive compound and an agent for a Photodynamic Therapy (PDT) containing zinc coproporphyrin-I useful for photodynamic therapy and a method for producing the same.

### Background Art

The photodynamic therapy is a therapeutic method that utilizes a photochemical reaction induced by a drug and a laser light and necrotizes cancer tissues by generating active oxygen in the cancer tissues.

Specifically, it is intended to cure diseases such as cancer and the like, by administering a cancer affinitive photosensitive drug to selectively accumulate it in cancer tissues, and by inducing a photochemical reaction by irradiating with a specific wavelength light the cancer tissues in which active oxygen and radicals are generated in the target tissues to necrotize cancer cells.

Compounds having a porphyrin skeleton have been known to be such photosensitive drugs, and these compounds are excited to a triplet state from a singlet ground state by irradiation of light having a wavelength of about 635 nm and necrotize cancer cells by generation of active oxygen and radicals.

Actually, Photofrin^{®} (Takeda Pharmaceutical Co. Ltd.) has been known to be such a photodynamic therapy drug to be used in such a therapy.

Photofrin^{®} is an oligomer (dimer to octamer) in which hematoporphyrin is bonded through ether and ester bonds. However, since it takes a long period of time (2-3 weeks in nude mice and HeLa cells) until Photofrin^{®} is excreted from the body of patients administered with this Photofrin^{®}, there was a problem that the patients treated with Photofrin^{®} can not be exposed to strong light, such as direct sunlight, and must stay in a place where light is scarce at least for one month until Photofrin^{®} is excreted from the patient's body.

Recently, a new photosensitive drug ATX-S10 (Na) was developed. For this case, the complete excretion period from the body of patients administered therewith is very short of only 7-8 hours, but the drug has a problem that photodynamic therapy must be performed at the same time or immediately after the administration of the photosensitive drug (non-patent document 1, 2).

In addition, it has been found that coproporphyrin-III produced by action of bacteria has a photosensitive activity, generates active oxygen by photo-irradiation and has a superior toxic effect on cancer cells compared to that of hematoporphyrin (patent document 1).

Also, zinc coproporphyrin-I has been found in meconium of a gravida but its photosensitivity, effect in PDT or the method for its industrial production is not known yet (non-patent document 3).

Further, heretofore, for these porphyrin metal complexes, it was not easy to insert transition metal ions to the porphyrin ring and there were limitations that the reaction shall be carried out at elevated temperatures under inert atmosphere and the like.

Thus, there has been a problem that porphyrin complexes having metal inserted in the porphyrin ring could not be synthesized more easily and under moderate conditions.

[Patent Document 1] Japanese Patent Publication Laid Open Specification No. 05-229948
[Non-Patent Document 1] M. Mori et al., Jpn. J. Cancer Res. 91. 845-852, 2000
[Non-Patent Document 2] Masumoto K. et al., Lasers Med Sci. 18. 134-138, 2003
[Non-Patent Document 3] K. Horiuchi et al., Clin. Chem. 37. 1173-1177, 1991
[Non-Patent Document 4] The 4th edition Jikken Kagaku Kouza Vol. 17, 385-395, 1991

### Disclosure of the Invention

### Problems to be Solved by the Invention

The present invention provides a novel photosensitive compound, a method for producing the same and a drug for photodynamic therapy containing the same.

### Means for Solving the Problems

The present invention is described as follows.
1. A compound represented by the following formula 1 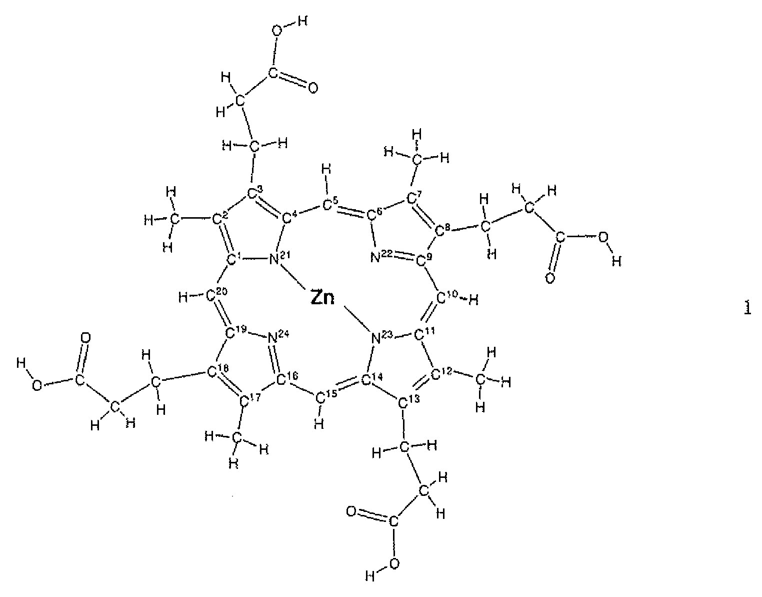 or pharmaceutically acceptable salts thereof.
2. A photodynamic therapy agent comprising the compound according to 1 described above.
3. A method for producing the compound according to 1 described above including the steps of:
   dissolving a compound represented by the following formula 2 in butanol;
   subjecting the butanol solution thus obtained to fractional extraction using an aqueous solution of ammonium acetate to obtain an aqueous solution of ammonium acetate salt containing the compound represented by the formula 2; and
   adding an aqueous solution of zinc chloride to the aqueous solution of ammonium acetate salt containing the compound represented by the formula 2. 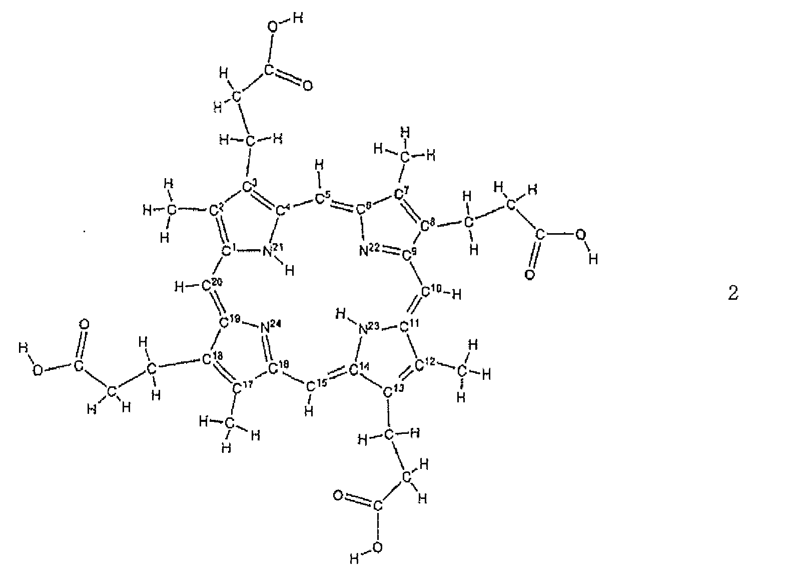
4. A method for producing the compound according to 1 described above, further including the steps of:
   adding the aqueous solution of the compound of the formula 1 to a PhenylSepharosea^{®} column pre-treated with the aqueous solution of ammonium acetate salt so that the compound of the formula 1 is retained to the column;
   washing the column using ammonium acetate;
   eluting the compound of the formula 1 using ultrapure water; and
   recovering the compound of the formula 1.

### [Advantages of the invention]

The present invention can be used suitably for photodynamic therapy, since the body retention time of the present invention is suitable for photodynamic therapy and it is inexpensive and safe.

### [Brief description of the Drawings]

Fig. 1A shows an absorption spectrum of zinc coproporphyrin-I. The vertical axis represents absorbance and the horizontal axis represents wavelength.
Fig. 1B shows an absorption spectrum of zinc coproporphyrin-I. The vertical axis represents absorbance and the horizontal axis represents wavelength.
Fig. 2 shows the in vivo pharmacokinetics of zinc coproporphyrin-I. The vertical axis represents relative values of fluorescence and the horizontal axis represents time after intravenous injection of zinc coproporphyrin-I to mice.

### Best Mode for Carrying Out the Invention

### A process for production of the compound of the formula 1

The compound of the formula 1 of the present invention can be produced according to the method shown below.
The starting material, the compound of the formula 2 (coproporphyrin-I), is marketed by Funakoshi Corp.

First, the compound of the formula 2 is dissolved in butanol, and the butanol solution thus obtained is subjected to a fractional extraction using an aqueous solution of ammonium acetate salt to obtain an aqueous solution of ammonium acetate salt containing the compound of the formula 2. The compound of the formula 1 can be obtained by adding an aqueous solution of zinc chloride to the aqueous solution of ammonium acetate salt containing the compound of the formula 2.

Since the compound of the formula 1 is photosensitive, the operations described above are preferably performed under light shielded conditions.

The drug of the present invention for photodynamic treatment contains the compound of the formula 1 or pharmaceutically acceptable salts thereof.

When the compound of the formula 1 and pharmaceutically acceptable salts thereof are administered to humans, it may be administered at the daily dosage of, for example, 50-100 mg/kg (body weight) per day, or preferably 150-300 mg/kg (body weight) per day in one or several times divided intravenous administrations. However, the administration dosage, frequency and route may be changed appropriately according to kind of diseases, symptoms, age, a route of administration and the like.

The compound of the formula 1 and pharmaceutically acceptable salts thereof may be orally administered in formulations such as tablets, capsules, granules, powder, syrups and the like, injected intraperitoneally or intravenously in injectable preparations or parenterally administered to the rectum in preparations such as a suppository. The content of the compound of the formula 1 or pharmaceutically acceptable salts thereof (active ingredient) may be varied from 1 to 90 weight %. For example, in the preparations of tablets, capsules, granules, powder and the like, the content of the active ingredient is preferably 5-80 weight %. In liquid formulations such as syrups, the content of the active ingredient is preferably 1-30 weight %. Further, in injectables for parenteral administrations, the content of the active ingredient is preferably 1-10 weight %.

Formulation of the compound of the formula 1 and pharmaceutically acceptable salts thereof can be prepared by a publicly known method using: formulation fillers (sugars such as lactose, white sugar, glucose, mannitol and the like; starches such as potato, wheat, corn and the like; inorganic substances such as calcium carbonate, calcium sulfate, sodium bicarbonate and the like; crystalline cellulose and the like); binders (starch paste, gum arabic, gelatin, sodium alginate, methylcellulose, ethylcellulose, polyvinylpyrrolidone, polyvinylalcohol, hydroxypropylcellulose, carmellose and the like); lubricants(magnesium stearate, talc, hydrogenated vegetable oil, macrogol, silicone oil); disintegrators (starch, agar, gelatin powder, crystalline cellulose, CMC.
Na, CMC.Ca, calcium carbonate, sodium bicarbonate, sodium alginate and the like); flavoring and aromatic agents (lactose, white sugar, glucose, mannitol, aromatic essential oils and the like); solvents (water for injections, sterilized purified water, sesame oil, soy bean oil, corn oil, olive oil, cotton seed oil and the like); stabilizers (inert gas such as nitrogen, carbon dioxide and the like, chelating agents such as EDTA, thioglycolic acid and the like, reducing agents such as sodium bisulfite, sodium thiosulfate, L-ascorbic acid, Rongalite^{®} and the like); preservatives (parahydroxybenzoate, chlorobutanol, benzylalcohol, phenol, benzalkonium chloride and the like); surface active agents (hydrogenated castor oil, polysorbate 80, 20 and the like); buffers (sodium salts of citric acid, acetic acid, phosphoric acid, boric acid and the like); diluents and the like.

The compound of the formula 1 and pharmaceutical salts thereof, for example, can break DNA efficiently under the irradiation of 350 nm light. Further, it was confirmed in an in vitro system using mice that these compounds do not demonstrate any anti-cellular activity by themselves but demonstrate a strong anti-cellular activity under the irradiation of light which induces DNA cleavage. Thus, after systemic administration of these compounds systemically, cancer tissues can be selectively killed, and the cancer can be treated without side effects by irradiating light only on the cancer tissues. Therefore, these compounds can be used as photodynamic therapy agents not only for surface cancer types (for example, breast cancer, skin cancer, esophageal cancer) but also deep cancer types (for example, gastric cancer, lung cancer).

### [Example 1]

The present invention will be practically described by Examples and Comparative Examples as follows. These Examples are to describe the present invention and do not limit the range of the present invention.

### Synthesis of the compound of the formula 1

A commercially available coproporphyrin-I· 2HCl (10 mg, 14 µmol) was dissolved in 300 ml 1-butanol. The solution thus obtained was transferred to a one liter separatory funnel and fractionally extracted with 4M ammonium acetate (twice with 300 ml and 200 ml). The combined 500 ml of the coproporphyrin-I ammonium acetate solution was mixed with 140 ml (14 mmol) of 1M zinc chloride solution to obtain crude zinc coproporphyrin-I aqueous solution (yield: 100%).

Next, 650 ml of crude zinc coproporphyrin-I aqueous solution was applied to a PhenylSepharose^{®} CL-4B column (ϕ4.1 x 38 mm, 500 ml) pre-equilibrated with 4 M ammonium acetate, and the column was further washed with 1.5 1 or more of 4 M ammonium acetate. Then, zinc coproporphyrin-I was eluted using ultrapure water, a colored band (dark red purple color) was recovered using a fraction collector to obtain purified zinc coproporphyrin-I (yield: 100%).

By similar procedures, zinc Photofrin^{®} and zinc ATX-S10 could be obtained from commercially available Photofrin^{®} and ATX-S10, respectively, (each with yield of 100%).

Aqueous solution of purified zinc coproporphyrin-I was applied to a SP Sephadex^{®} (Na type cationic ionexchange resin) column and eluted with ultrapure water. The eluate was lyophilized to obtain purified zinc coproporphyrin-I (Na') (yield: 100%).

### [Example 2]

### Measurement of Photophysical Characteristics of zinc coproporphyrin-I

### Absorption

### Experimental Results

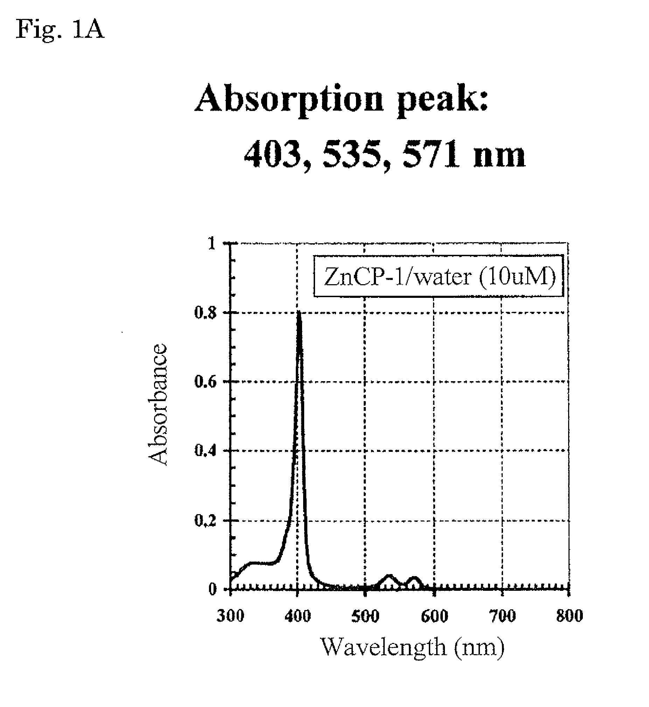

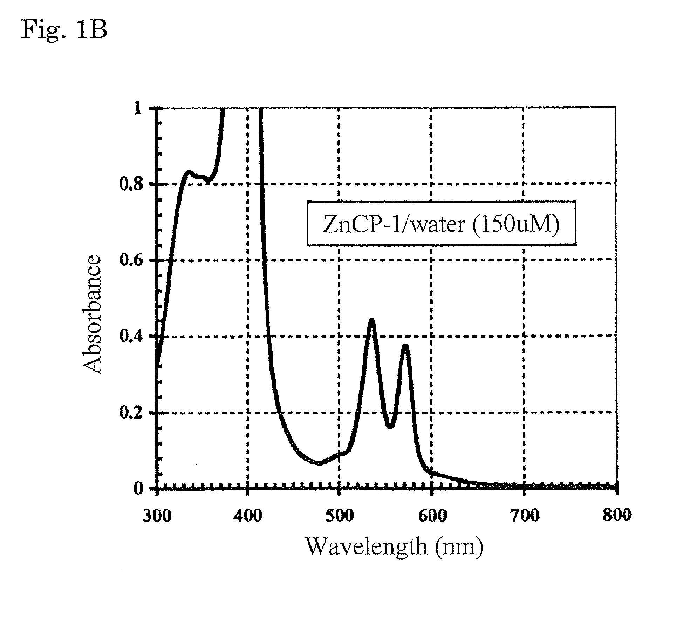

The absorption spectra of zinc coproporphyrin-I are shown in Fig. 1 A and B. The absorption peaks were observed at 403, 535 and 571 nm.
Also, the generation of singlet oxygen was confirmed with excitation at 563 nm and emission at 1270 nm in near infrared light.

### [Example 3]

### In vivo Pharmacokinetics of zinc coproporphyrin-I

### Experimental Method

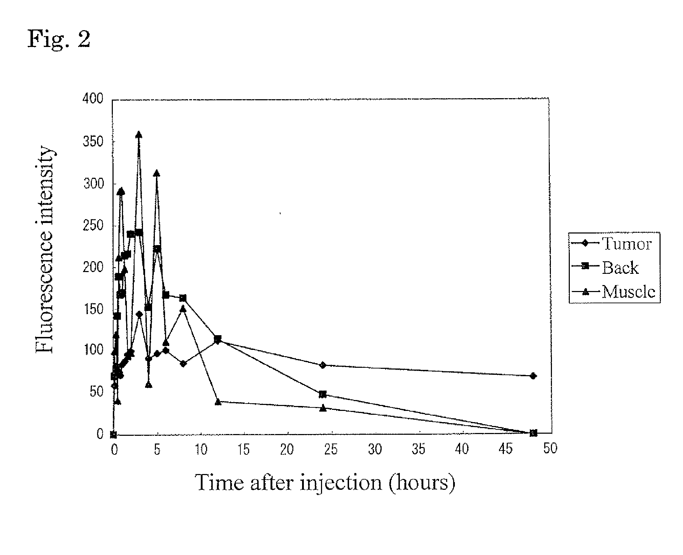

In the back of a (BALB/c) nude mouse, 5 x 10⁶ HeLa cells in 0.1 ml were transplanted intradermally. When the tumor grew to 12-17 mm diameter, 25 mg/kg of zinc coproporphyrin-I in physiological saline was intravenously injected, and mice were sacrificed at each time shown in Fig. 2, and then zinc coproporphyrin-I in 3 kinds of tissues (tumor, back and muscle) was measured with excitation at 405 nm and fluorescence at 580 nm.

### Experimental Results

Zinc coproporphyrin-I was excreted out of the body in 3-4 days (Fig. 2).

### [Example 4]

### Drug efficacy of the agent for photodynamic therapy Experimental Method

To the back of a (BALB/c) nude mouse, 5 x 10⁶ HeLa cells in 0.1 ml were intradermally transplanted. When the tumor grew to 12-17 mm diameter, 203 mg/kg of zinc coproporphyrin-I in physiological saline was intravenously injected intravenously, and after 28 minutes mice were irradiated repeatedly with YAG-Dye laser at a frequency of 30 Hz, with 80 mV/cm² and total irradiation dose of 100 J/cm². The wavelength was 580 nm. The light was emitted horizontally to the side of the tumor. To observe the necrosis layer, the tumors were excised out at 24 hours after the laser irradiation, fixed in 20% formaldehyde solution and embedded in paraffin. Sections were prepared, stained with HE and observed.

### Results

### Observations

Light irradiation to the HeLa tumor resulted in thrombus formation in the capillary vessels and hemorrhage inside of the tissues. For HeLa cells, anucleated cells, chromatin condensed cells and cells with ruptured vacuoles were seen, and cells appearing to be dead were observed in about 10% of the total cells.

### Industrial Applicability

The compounds of the present invention are useful for photodynamic therapy.

## Claims

1. A compound represented by the following formula 1 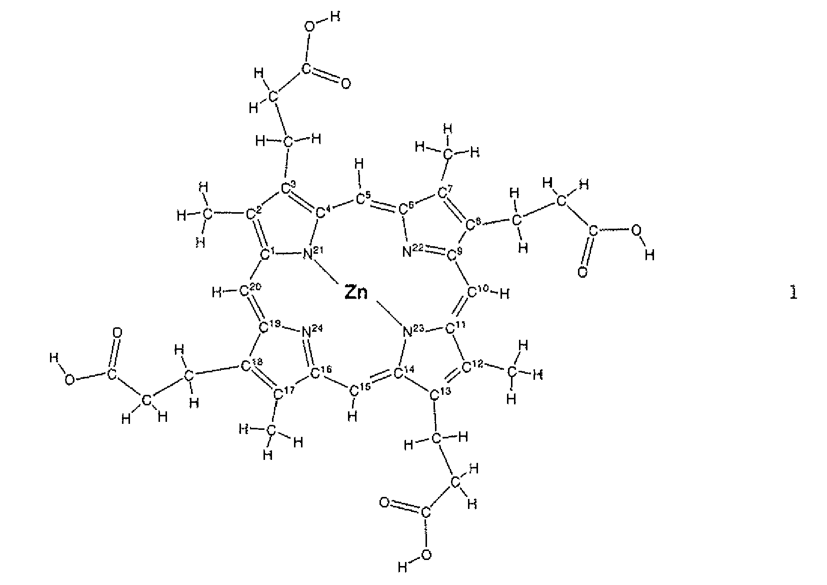 or pharmaceutically acceptable salts thereof.

2. A photodynamic therapy agent comprising the compound according to claim 1.

3. A method for producing the compound according to claim 1 comprising the steps of:
dissolving a compound represented by the following formula 2 in butanol; 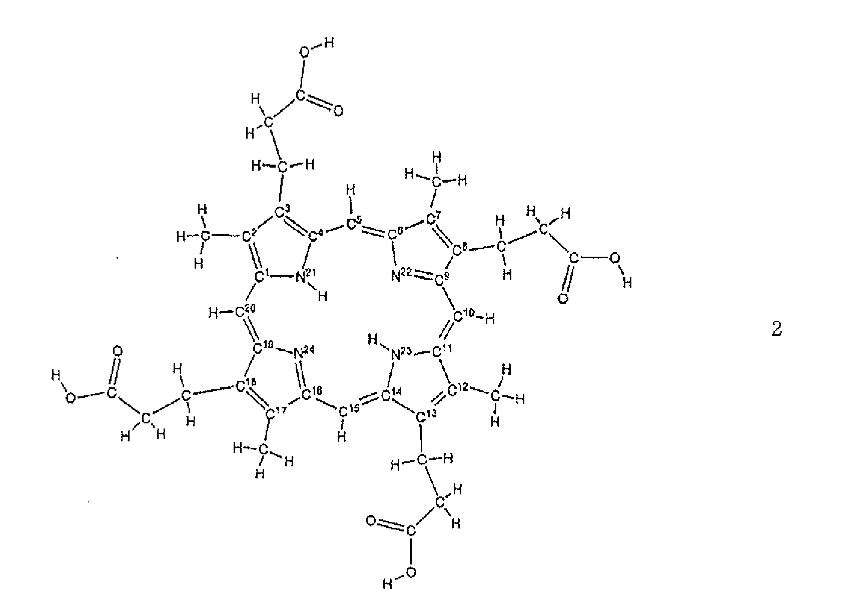
subjecting the butanol solution obtained to fractional extraction using an aqueous solution of ammonium acetate to obtain an aqueous solution of ammonium acetate salt containing the compound represented by the formula 2; and
adding an aqueous solution of zinc chloride to the aqueous solution of ammonium acetate salt containing the compound represented by the formula 2.

4. The method for producing the compound according to claim 1, further comprising the steps of:
adding the aqueous solution of the compound of the formula 1 to a PhenylSephaxose^{®} column pre-treated with the aqueous solution of ammonium acetate salt so that the compound of the formula 1 is retained to the column;
washing the column using ammonium acetate;
eluting the compound of the formula 1 using ultrapure water; and
recovering the compound of the formula 1.
